# EUROPÄISCHE PATENTSCHRIFT

(11) **EP 2 685 885 B1**
(45) Veröffentlichungstag und Bekanntmachung des Hinweises auf die Patenterteilung: **26.08.2015**
(21) Anmeldenummer: 12756645.3
(22) Anmeldetag: 05.06.2012
(51) Int. Cl.: A61B 5/0205, A61B 5/16

(54) **STRESS- UND BURNOUTANALYSE- UND DIAGNOSEGERÄT**
STRESS AND BURN-OUT ANALYSIS AND DIAGNOSTIC DEVICE
APPAREIL D'ANALYSE ET DE DIAGNOSTIC DU STRESS ET DE L'ÉPUISEMENT PROFESSIONNEL

(43) Veröffentlichungstag der Anmeldung: 22.01.2014
(73) Patentinhaber: Beurer GmbH, 89077 Ulm (DE)
(72) Erfinder: KOUEMOU, Guy Leonard, 89081 Ulm (DE)
(74) Vertreter: Hentrich Patentanwälte PartG mbB
(86) Internationale Anmeldenummer: PCT/DE2012/100169
(87) Internationale Veröffentlichungsnummer: WO 2013/182173

(56) Entgegenhaltungen:
- EP-A2- 2 014 225
- US-A1- 2007 173 901
- US-A1- 2009 069 641
- US-A1- 2010 240 982
- US-A1- 2011 166 430
- US-A1- 2012 136 226
- Anonymous: "Fire of life", Primedica , 2011, XP002686887, Gefunden im Internet: URL:http://www.primedica.de/fire-of-life [gefunden am 2012-11-12]
- VAN LUIJTELAAR GILLES ET AL: "EEG findings in burnout patients.", THE JOURNAL OF NEUROPSYCHIATRY AND CLINICAL NEUROSCIENCES SPRING 2010, Bd. 22, Nr. 2, April 2010 (2010-04), Seiten 208-217, XP002686888, ISSN: 1545-7222
- W DE VENTE: "Physiological differences between burnout patients and healthy controls: blood pressure, heart rate, and cortisol responses", OCCUPATIONAL AND ENVIRONMENTAL MEDICINE, Bd. 60, Nr. >90001, 1. Juni 2003 (2003-06-01), Seiten 54i-61, XP055043801, ISSN: 1351-0711, DOI: 10.1136/oem.60.suppl_1.i54

## Beschreibung

Die Erfindung betrifft ein Analyse- und Diagnosegerät zur Detektion von Stress bei einem Probanden mit mindestens einem Vitalsignale des Probanden detektierenden und diese einer Speichereinheit zuführenden Sensor (2), einer Spannungsversorgung, sowie mit einer mit der Speichereinheit verbundenen Auswerteeinheit, wobei mittels der Auswerteeinheit ein zeitaufgelöster Verlauf der durch den mindestens einen Sensor (2) detektierten Vitalsignale auswertbar ist, und wobei in der Auswerteeinheit ein mittels eines Abgleichs des zeitaufgelösten Verlaufs der detektierten Vitalsignale mit in einer Datenbank gespeicherten zeitaufgelösten Stress -Vitalmustern Stress diagnostizierender Komparator vorgesehen ist.

Durch Stress kann das Burnout-Syndrom hervorgerufen werden. Nach einer Schätzung leiden bis zu 25% aller Erwerbstätigen unter Burnout, wobei nicht alle gerade deshalb krankgeschrieben werden. Vielmehr wird ein Großteil wegen der durch das Burnout-Syndrom hervorgerufenen Begleitkrankheiten krankgeschrieben. Das Burnout-Syndrom selbst wird dabei selten ebenfalls diagnostiziert. Das Burnout-Syndrom oder das sogenannte "Ausgebranntsein" schlägt sich in typischen Symptomen, wie Schlafstörungen, depressiven Verstimmungen, ständiger Nervosität und in Konzentrationsstörungen nieder. Für die Betroffenen ist es dabei häufig nicht möglich während des Schlafes eine Regeneration des Körpers und der Psyche zu erzielen. Negativer Dauerstress gehört laut Weltgesundheitsorganisation (WHO) zu den größten Gesundheitsrisiken des 21. Jahrhunderts, weswegen das Burnout-Syndrom oder das Ausgebranntsein als ein den Gesundheitszustand beeinflussender Faktor in die Internationale statistische Klassifikation der Krankheiten und verwandter Gesundheitsprobleme (ICD-10) klassifiziert ist.

Das gängigste Hilfsmittel, um das Burnout-Syndrom festzustellen, ist das von Christina Maslach und Susan E. Jackson entwickelte Maslach Burnout Inventory (MBI). Dieses besteht aus einem Fragenkatalog mit insgesamt 22 Fragen, in dem eine der drei Ausprägungen des Burnout Syndroms festgestellt werden soll. Diese drei Dimensionen des Burnout-Syndroms bestehen nach dem MBI aus "Depersonalisierung", aus "Emotionaler Erschöpfung", und aus "Erleben von Misserfolg". Die Burnout-Dimension der "Depersonalisierung" zeigt sich dabei insbesondere in Symptomen wie Gleichgültigkeit, wie Zynismus und Distanz. Die zweite Burnout-Dimension "Emotionale Erschöpfung" schlägt sich in den Symptomen Reizbarkeit, ständige Anspannung und ständige Antriebsschwäche nieder. Die Burnout-Dimension "Erleben von Misserfolg" ist symptomatisch gekennzeichnet durch Sinnentleerung, durch Unwirksamkeit und durch Hyperaktivität.

Darüber hinaus sind weitere Testverfahren gegeben durch das Copenhagen Burnout Inventory (CBI), durch das Oldenburg Burnout Inventar (OLBI), das Hamburger Burnout Inventar (HBI), durch das Shirom-Melamed Burnout Questionnaire (SMBQ) und durch das School- Burnout-Inventory (SBI), welche sich alle im Kern jedoch nicht von ihrem Vorreiter, dem Maslach Burnout Inventory (MBI) unterscheiden.

Allen gemein ist, dass diese aus Fragebögen oder Fragenkatalogen bestehen, welche der Patient durch sein subjektives Empfinden zu beantworten hat, und woraus vom entsprechenden Facharzt erst nach einer Auswertung der Fragebögen eine Diagnose getroffen werden kann.

Aus der US 2009 / 0 069 641 A1 ist bereits ein System zur Analyse von Stress bekannt. Mit diesem Gerät werden Vitalsignale erfasst und mit Werten aus einer Datenbank verglichen, um festzustellen, welcher Grad an Stress vorliegt. Zur Ermittlung des Grades an Stress wird die Herzratenvariabilität bestimmt, womit also die Herzrate gemessen wird. Dieses System weist den Nachteil auf, dass einerseits keine strömende Atemluft erfasst werden kann und andererseits der Proband oder Patient nicht aktiv auf eine ggfs. vorliegende Stresssituation hingewiesen werden kann.Dieses System wird des Weiteren nicht im Heimbereich bzw. Konsumentenbereich eingesetzt.

Es ist daher die Aufgabe in der vorliegenden Erfindung ein Analyse- und Diagnosegerät zur Detektion von Stress bereit zu stellen, das eine verbesserte Diagnose von Stress erlaubt.

Die Aufgabe wird bei einem Gerät der eingangs genannten Art dadurch gelöst, dass der Sensor als ein bioakustischer Sensor zur Detektion intrakorporal erzeugten Schalls gebildet ist, wobei eine Alarmeinheit vorgesehen ist zur Alarmierung des Probanden oder Patienten im Falle einer Stresssituation.

Mit der Erfindung ist der Vorteil verbunden, dass nunmehr ein quantitatives Messverfahren zur Beschreibung von Stress und eines Burnout-Syndroms genutzt werden kann, und dass eine qualitative Methode zur Beschreibung des aktuellen Befindens eines Menschen bezüglich Stress und/oder des Burnout-Syndroms geschaffen ist. Außerdem kann durch den bioakustischen Sensor der Herzschlag und die Atemflussgeräusche erfasst werden.

Durch die Alarmeinheit kann der Proband oder Patient alarmiert werden, wenn er in eine Stresssituation bzw. Burnoutsituation gerät. Somit stellt der Einsatz einer Alarmeinheit eine Möglichkeit dar, den Probanden mit einem Burnoutsyndrom bzw. mit Dauerstress zu therapieren. Diese Alarmeinheit kann dabei auf unterschiedlichste Weise gebildet sein.

Die Alarmeinheit kann als ein Elektrostimulator, nämlich in Form von transkutaner elektrischer Nervenstimulation (TENS) und/oder elektrischer Muskelstimulation (EMS), als ein Vibrator, als ein Schallgeber, als eine Signalleuchte, als ein Temperaturreizgeber, als ein Geruchssignalgeber, als eine mechanische Schlageinheit, als eine Pickeinheit oder als eine Stoßeinheit gebildet sein. Auch kann die Alarmeinheit aus einer Kombination der vorgenannten Mittel zur Alarmierung gebildet sein.

Die Alarmeinheit kann an einem Arm- und/oder einem Beinband befestigt sein, oder auch an dem Gurtsystem befestigt oder aber auch in dieses integriert sein. Hierdurch lässt sich das erfindungsgemäße Gerät sehr kompakt bilden.

Die Stress- und Burnout-Vitalmuster, die in der Datenbank hinterlegt sind können dabei hinsichtlich des Alters, des Geschlechts, und hinsichtlich der aktuellen Tätigkeit des Probanden oder Patienten variieren. Insbesondere können auch Daten hinterlegt werden, die Daten aus momentan in der Wissenschaft verwendeten Standardfragebögen zur Bemessung von Stress und/oder des Burnout-Syndroms enthalten.

Besonders günstig ist es, wenn die die Stress- und Burnout-Vitalmuster enthaltende Datenbank in der Speichereinheit vorgesehen ist, denn so kann das Analyse- und Diagnosegerät den Probanden oder Patienten direkt darüber informieren, ob die gemessenen Vitalsignale mit denjenigen der Stress- und Burnout-Vitalmustern in der Speichereinheit übereinstimmen. Dies ist eine große Erleichterung für den Patienten, denn nun kann er das Gerät auch einfach zuhause verwenden und das Analyse- und Diagnosegerät ist somit im Homepflegebereich einsetzbar.

Alternativ ist es aber auch sinnvoll, wenn die die Stress- und Burnout-Vitalmuster enthaltende Datenbank in einem externen, über ein Netzwerk zugänglichen Rechen- und Speicherwerk vorgesehen ist. So kann die Datenbank also in einem häufig als "Cloud" bezeichneten Serverpark hinterlegt sein, von wo aus sie für eine Vielzahl von Probanden oder Patienten mit ihren Analyse-und Diagnosegeräten zugänglich ist. Dem Patienten oder Probanden kann auf diese Weise einfacher neue, Stress und/oder das Burnout-Syndrom indizierende Stress- und Burnout-Vitalmuster zur Verfügung gestellt werden, die eine Auswertung für die Auswerteeinheit erleichtern und beschleunigen.

Besonders vorteilhaft ist es, wenn eine Sensoranordung vorgesehen ist, die den bioakustischen Sensor, sowie mindestens einen weiteren Sensor aufweist, der aus der Gruppe bestehend aus einem Gasflusssensor zur Messung des Gasflusses der Atmung, einem bioakustischen Sensor zur Messung der Probandengeräusche, einem Hautimpendanzsensor, einem EKG-Sensor, einem Pulsoximetriesensor, einem EOG-Sensor und einer Einheit zur Messung des Blutdrucks ausgewählt ist.

Mittels eines Gasflusssensors, oder einem bioakustischen Sensor, oder einem Pulsoximetriesensor, oder einem EOG-Sensor (Sensor für die Elektrookulografie) lässt sich die Schlafqualität des Probanden messen. Hierbei kann detektiert werden, ob beispielsweise ein Schlafapnoe-Syndrom vorhanden ist und ob die Anzahl der Schlafapnoen pro Nacht gesund oder darüber informieren, ob die gemessenen Vitalsignale mit denjenigen der Stress- und Burnout-Vitalmustern in der Speichereinheit übereinstimmen. Dies ist eine große Erleichterung für den Patienten, denn nun kann er das Gerät auch einfach zuhause verwenden und das Analyse- und Diagnosegerät ist somit im Homepflegebereich einsetzbar.

Alternativ ist es aber auch sinnvoll, wenn die die Stress- und Burnout-Vitalmuster enthaltende Datenbank in einem externen, über ein Netzwerk zugänglichen Rechen- und Speicherwerk vorgesehen ist. So kann die Datenbank also in einem häufig als "Cloud" bezeichneten Serverpark hinterlegt sein, von wo aus sie für eine Vielzahl von Probanden oder Patienten mit ihren Analyse-und Diagnosegeräten zugänglich ist. Dem Patienten oder Probanden kann auf diese Weise einfacher neue, Stress und/oder das Burnout-Syndrom indizierende Stress- und Burnout-Vitalmuster zur Verfügung gestellt werden, die eine Auswertung für die Auswerteeinheit erleichtern und beschleunigen.

Besonders vorteilhaft ist es, wenn der mindestens eine Sensor aus der Gruppe bestehend aus einem Gasflusssensor zur Messung des Gasflusses der Atmung, einem bioakustischen Sensor zur Messung der Probandengeräusche, einem Hautimpendanzsensor, einem EKG-Sensor, einem Pulsoximetriesensor, einem EOG-Sensor und einer Einheit zur Messung des Blutdrucks ausgewählt ist.

Mittels eines Gasflusssensors, oder einem bioakustischen Sensor, oder einem Pulsoximetriesensor, oder einem EOG-Sensor (Sensor für die Elektrookulografie) lässt sich die Schlafqualität des Probanden messen. Hierbei kann detektiert werden, ob beispielsweise ein Schlafapnoe-Syndrom vorhanden ist und ob die Anzahl der Schlafapnoen pro Nacht gesund oder ungesund ist. Es lässt sich mit dieser Messmethode auch der Anteil der Tiefschlafphasen pro Nacht ableiten. Der Anteil des Tiefschlafes bei einem gesunden Menschen beträgt 20 bis 25%. Ist der gemessene Tiefschlafphasen-Anteil dagegen signifikant geringer, so lässt sich daraus eine Schlafstörung ableiten, die als ein Beitrag für Stress und/oder das Burnout-Syndrom erkannt werden kann.

Eine weitere Auswertung ist beispielsweise mit einem Gasflusssensor zur Messung des Gasflusses der Atmung und auch mit dem bioakustischen Sensor zur Messung der Probandengeräusche möglich, mit denen dabei die Atemfrequenz gemessen wird. Ein gesunder Erwachsener macht normalerweise 11 bis 15 Atemzüge pro Minute, eine Abweichung hiervon kann auf Stress und/oder ein Burnout-Syndrom hindeuten. Ganz häufig ist Stress und/oder das Burnout-Syndrom durch eine seelisch bedingte Dyspnoe gekennzeichnet, welche sich durch ein - evtl. anfallsweise - auftretendes tiefes Atemholen bemerkbar macht. Der Stress- und Burnout-Patient verspürt den Zwang tief einzuatmen. Diese sogenannte "Seufzeratmung" kann bei verschiedenen Krankheitsbildern mit Beeinflussung der höhergradigen Atemzentren auftreten, zu denen unter anderem das obstruktive Schlafapnoesesyndrom zählt.

Desweiteren kann die Herzfrequenz des Probandenherzen mit einem EKG-Sensor oder mit dem bioakustischen Sensor gemessen und die erfassten Vitalsignale in der Auswerteeinheit zeitaufgelöst ausgewertet werden. Bei einem sich in Ruhe befindenden, herzgesunden und normal trainierten Erwachsenen sind 60 bis 80 Herzschläge pro Minute die Regel. Eine Abweichung von diesen Normalwerten kann bereits Stress- und/oder ein Burnout-Syndrom indizieren. Selbstverständlich ist diese Herzfrequenz bei Sportlern niedriger, was bei der Auswertung der Messdaten und dem Abgleich mit den Stress- und Burnout-Vitalmustern zu berücksichtigen ist.

Mit einer Einheit zur Messung des Blutdrucks kann dieser untersucht werden, ob er im normalen Bereich von systolischem Druck von 120 mm Hg und von diastolischem Druck von 80 mm Hg liegt, oder ob er von diesen Normalparametern abweicht. Besonders hoher diastolischer Druck lässt auf ein "Gestresstsein" schließen, so dass auch hierin eine Gefahr des Ausbruchs des Burnout-Syndroms bestehen kann.

Mit einem Hautimpendanzsensor kann die Hautimpendanz gemessen werden und in der Auswerteeinheit die Hautimpendanz-Variation ausgewertet werden. Mit dem Hautimpedanzsensor lässt sich die sogenannte elektrodermale Aktivität erfassen, die ein kurzzeitiges Absinken des elektrischen Leitungswiderstandes der Haut bewirkt, durch die typische Erhöhung des Sympathikotonus bei emotional-affektiven Reaktionen. Durch die dabei entstehende erhöhte Schweißsekretion nimmt die Leitfähigkeit der Haut zu. Es lassen sich also durch die Messung der elektrodermalen Aktivitäten psychophysiologische Zusammenhänge objektivieren, weshalb jede physiologische Erregung, wie sie mit Emotionen oder mit Stress einhergeht, die Hautleitfähigkeit verändert.

Außerdem lässt sich mittels des EKG-Sensors die Herzratenvariabilität auswerten, die durch Einflussgrößen wie Alter, Geschlecht, Atmung und Trainingszustand des Probanden oder Patienten bestimmt ist. Eine verminderte Herzfrequenz-Variabilität weist auf eine gesteigerte Gefährdung hin, einen Herzinfarkt zu bekommen.

Es ist weiterhin bevorzugt, wenn wenigstens zwei der Sensoren aus der vorstehend genannten Gruppe vorgesehen sind, da nunmehr auch eine Kombination der vorgenannten Parameter messbar und auswertbar ist. Durch den Abgleich eines Bündels an Vitalsignalen mit einem entsprechenden Bündel an Stress- und Burnout-Vitalmustern lässt sich Stress und/oder das Burnout-Syndrom noch einfacher und verlässlicher feststellen und diagnostizieren.

Von Vorteil ist weiterhin, wenn mindestens ein Bedienelement vorgesehen ist, welches vorzugsweise als ein Einknopf-Bedienelement gebildet ist. Dieses Bedienelement kann dem Ein- bzw. Ausschalten des Gerätes dienen, wobei desweiteren eine Kalibrierung durch beispielsweise längeres Drücken des Gerätes erfolgt. Durch die Bildung als ein Einknopf-Bedienelement bleibt das Analyse- und Diagnosegerät kompakt, wobei das Bedienelement als ein einfacher Dreh-, Schiebe-, Tast- oder sonstiger aus dem Stand der Technik bekannter Schalter gebildet sein kann.

Weiterhin ist es besonders günstig, wenn eine Anzeigevorrichtung vorgesehen ist, und wenn die Anzeigevorrichtung geeignet ist, das Diagnoseergebnis der Auswerteeinheit darzustellen. Hierdurch ist gewährleistet, dass der Patient schnell und einfach darüber informiert ist, ob Stress und/oder ein Burnout-Syndrom vorliegt oder nicht. Dabei ist vorteilhaft, wenn die Anzeigevorrichtung gleichwohl als ein Eingabeterminal gebildet ist, das vorzugsweise als Tastschirm gebildet ist. Durch dieses Eingabeterminal kann der Proband oder Patient seine Grundparameter wie beispielsweise Alter, Gewicht, Geschlecht, Tätigkeit selbst eingeben, womit die Auswertung für die Auswerteeinheit vereinfacht ist.

Sinnvoll ist es auch, wenn mindestens eine Schnittstelle zur Dateneingabe und/oder zur Datenausgabe vorgesehen ist, denn auf diese Weise können die Messdaten auch auf einem externen Gerät dargestellt bzw. weitergehend ausgewertet werden. Als Schnittstelle kann dabei z.B. ein USB-Anschluss, ein Local-Area-Network-Anschluss (LAN-Anschluss) oder ein in sonstiger Weise gebildeter und aus dem Stand der Technik bekannter Anschluss vorgesehen sein. Auch kann eine drahtlose Schnittstelle in Form von WLAN, Bluetooth, oder in anderer bekannter Form vorgesehen sein. Durch diese Schnittstelle ist es weiterhin möglich für den Probanden oder Patienten eine Fern- oder Telemedizin bereitzustellen, bei der der Arzt nicht mehr direkt vor Ort sein muss, sondern an seinem entfernt gelegenen Bildschirm und Computer die Daten des Analyse- und Diagnosegeräts des Patienten einsehen kann.

Außerdem ist vorteilhaft, wenn die Auswerteeinheit als ein Bauteil gebildet ist, welches aus der Gruppe bestehend aus einem Mikrocontroller, einem ASIC (anwendungsspezifisch integrierte Schaltung), einem FPAA (feldprogrammierbare Analoganordnung) und einem FPGA (feldprogrammierbare Gatter-Anordnung) ausgewählt ist. Hierdurch kann die Auswerteeinheit besonders klein und kompakt gebildet werden, wodurch das Analyse- und Diagnosegerät selbst nur noch sehr geringe Abmessung aufweisen muss.

Bevorzugt ist es, wenn dem Mikrocontroller mindestens ein Frequenzfiltersystem zugeordnet ist. Hierdurch können beispielsweise die die Atemgeräusche überdeckenden Raum- und Umgebungsgeräusche herausgefiltert werden, wobei in einer bevorzugten Ausgestaltung das Frequenzfiltersystem als ein ein- oder mehrkanaliger, hard- und/oder softwarebasierter Filter gebildet ist.

Desweiteren ist günstig, wenn ein mit der Auswerteeinheit verbundener interner oder externer Analog-Digital-Wandler vorgesehen ist, da auf diese Weise eine sehr schnelle Auswertung der Daten und ein sehr schneller Abgleich der Daten, d.h. der Vitalsignale und der Burnout-Vitalmuster, durchgeführt werden kann.

Um darüber hinaus den mindestens einen Sensor an vorbestimmte Positionen am Probandenkörper befestigen zu können, ist es sinnvoll, wenn ein Gurtsystem zur Lagefixierung des mindestens einen Sensors vorgesehen ist.

Auch kann das Analyse- und Diagnosegerät sehr kompakt gebildet werden, wenn der mindestens eine Sensor in das Gurtsystem integriert ist. Außerdem wird vermieden, dass unnötig viele Kabel zu den Sensoren am Probandenkörper verlaufen, die ihn während der Messung in seinem Alltag oder seinem Schlafbefinden einschränken oder behindern.

Letztlich ist es auch bevorzugt, wenn das Gurtsystem aus textilem Material, vorzugsweise elastisch gebildet ist. Textile Materialien fühlen sich auf der Haut angenehmer an, als beispielsweise Kunststoffmaterialien, so dass das Gurtsystem für den Probanden sehr angenehm zu tragen ist. Darüber hinaus sind textile Materialien optimal, wenn Schweißbildung, insbesondere bei langem Tragen auftritt. Bevorzugt und besonders günstig ist es daher, wenn das Gurtsystem abnehmbar und waschbar ist. Diese Reinigungsmöglichkeit bietet hygienische Vorteile für den Probanden. Darüber hinaus ist es sinnvoll, wenn das Gurtsystem längenverstellbar gebildet ist und zur Festlegung der Länge dienende Schließen, beispielsweise in Form von Schnallen oder anderen Befestigungsmitteln aufweist.

Im Folgenden wird die Erfindung an einem in der Zeichnung dargestellten Ausführungsbeispiel näher erläutert; es zeigt:

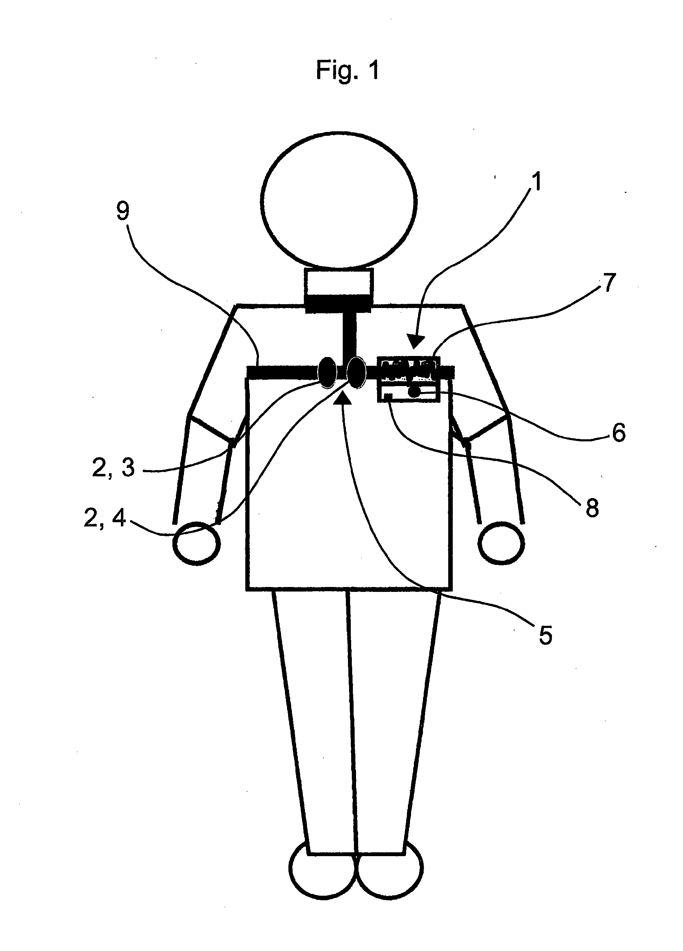

- Fig. 1: das am Probanden angelegte Analyse- und Diagnosegerät.

In Fig. 1 ist das Analyse- und Diagnosegerät 1 zur Detektion von Stress und des Burnout-Syndroms bei einem Probanden gezeigt. Dieses weist mindestens einen Vitalsignale des Probanden detektierenden und diese einer Speichereinheit zuführenden Sensor 2, eine Spannungsversorgung, sowie eine mit einer Speichereinheit verbundene Auswerteeinheit auf. Mittels der Auswerteeinheit ist ein zeitaufgelöster Verlauf der durch den mindestens einen Sensor 2 detektierten Vitalsignale auswertbar. In der Auswerteeinheit ist außerdem ein mittels eines Abgleichs des zeitaufgelösten Verlaufs der detektierten Vitalsignale mit in einer Datenbank gespeicherten zeitaufgelösten Stress- und Burnout-Vitalmustern Stress und das Burnout-Syndrom diagnostizierender Komparator vorgesehen.

Im Ausführungsbeispiel ist die die Stress- und Burnout-Vitalmuster enthaltende Datenbank in der Speichereinheit vorgesehen. Außerdem sind genau zwei der Sensoren 2 in einer Sensoranordnung 5 vorgesehen.

Die Sensoren 2 der Sensoranordnung 5 sind ausgewählt aus der Gruppe bestehend aus einem Gasflusssensor zur Messung des Gasflusses der Atmung, einem bioakustischen Sensor 3 zur Messung der Probandengeräusche, einem Hautimpendanzsensor 4, einem EKG-Sensor, einem Pulsoximetriesensor und einer Einheit zur Messung des Blutdrucks.

Im gezeigten Ausführungsbeispiel ist ein außerhalb des Gerätegehäuses angeordneter bioakustischer Sensor 3 zur Geräuscherkennung vorgesehen, welcher die Vitalsignale erfasst und diese drahtgebunden oder drahtlos der Speichereinheit zuführt. Der Sensor 2 kann selbstverständlich auch innerhalb des Gehäuses angeordnet sein. Der in diesem Ausführungsbeispiel gezeigte bioakustische Sensor 3 detektiert intrakorporal, also innerhalb des Probandenkörpers erzeugten Schall. Aber auch Umgebungsgeräusche können durch ihn detektiert werden. Der bioakustische Sensor 3 ist im Ausführungsbeispiel torsonah, also in der Nähe des oder direkt am Torso des Probanden angeordnet und misst die Geräusche, die durch den Luftfluss in der Luftröhre zu Stande kommen. Er kann aber auch die Geräusche des Herzschlags erfassen. Es können selbstverständlich auch alle dieser Sensoren 2 in der Sensoranordnung 5 genutzt werden.

Desweiteren besitzt die Sensoranordnung 5 des Ausführungsbeispiels einen Hautimpendanzsensor 4, der die Leitfähigkeit der Haut des Probanden misst. Somit ist folglich eine Bündelmessung des Datenbündels bestehend aus akustischen Atemfluss-Vitalsignalen und bestehend aus Hautleitfähigkeits-Vitalsignalen ermöglicht.

Das gezeigte Analyse- und Diagnosegerät weist 1 ein Gurtsystem 9 zur Lagefixierung am Probandenkörper der beiden Sensoren 2 der Sensoranordnung 5 auf. Der Hautimpendanzsensor 4 ist dabei in das Gurtsystem 9 integriert, welches hier aus einem textilen Material gebildet ist. Dieses ist desweiteren längenverstellbar. An dem Gerätegehäuse ist ein als ein Einknopfbedienelement gebildetes Bedienelement 6 angeordnet, das unter anderem dem Ein- bzw. Ausschalten des Analyse- und Diagnosegeräts dient.

Es ist außerdem eine Anzeigevorrichtung 7 vorgesehen, die geeignet ist, das Diagnoseergebnis der Auswerteeinheit darzustellen. Das Analyse- und Diagnosegerät 1 des Ausführungsbeispiels weist eine Schnittstelle 8 zur Datenein- und zur Datenausgabe auf, welche hier als USB-Schnittstelle gebildet ist. Im Gehäuseinneren ist die Auswerteeinheit angeordnet, die als ein Mikrocontroller gebildet ist, welchem ein Frequenzfiltersystem zur Filterung von bestimmten Frequenzen zugeordnet ist. Dieses Filtersystem filtert Störgeräusche und Geräuschartefakte heraus, welche vom bioakustischen Sensor 3 detektiert wurden. Das Filtersystem ist vorzugsweise als ein Hochpass-Filtersystem gebildet.

Nachfolgend soll erläutert werden, wie die Detektion von Stress und/oder des Burnout-Syndroms mit dem erfindungsgemäßen Analyse- und Diagnosegerät 1 aus Fig. 1 erfolgt.

In der Datenbank sind Parameter des Probanden, nämlich Alter, Geschlecht und seine aktuelle Tätigkeit hinterlegt. Desweiteren sind Burnout-Vitalmuster gespeichert, nämlich akustische Stress- und Burnout-Vitalmuster und Stress- und Burnout-Vitalmuster betreffend die Hautimpendanz. Der Proband trägt das Analyse- und Diagnosegerät 1 drei bis vier Tage und Nächte am Körper, wobei der bioakustische Sensor 3 und der Hautimpendanzsensor 4 permanent oder auch in regelmäßigen oder unregelmäßigen Zeitintervallen Vitalsignale detektieren und diese in der Speichereinheit ablegen. Es ist zur Diagnostik auch möglich das Analyse- und Diagnosegerät 1 regelmäßig über einen kürzeren Zeitraum am Probanden anzulegen.

Der bioakustische Sensor 3 detektiert die Atemflussgeräusche während des Schlafes und bemisst anhand dieser Vitalsignale die Schlafqualität, wobei kontrolliert wird, ob ein Schlafapnoesyndrom vorhanden ist. Außerdem kontrolliert er während der Wachphasen des Probanden, ob eine seelisch bedingte Dyspnoe, also eine Seufzeratmung in regelmäßigen Abständen auftritt. Die Häufigkeit oder die Frequenz der Seufzeratmungen des Probanden wird durch die Auswerteeinheit in einem zeitaufgelösten Verlauf festgehalten. Dieser zeitaufgelöste Verlauf der mit dem bioakustischen Sensor 3 detektierten Vitalsignale wird durch die Auswerteeinheit mit den in der Datenbank gespeicherten, zeitaufgelösten Stress- und Burnout-Vitalmustern verglichen, wobei die Auswerteeinheit das Vorliegen von Stress und/oder des Burnout-Syndroms erkennt, wenn eine Übereinstimmung zwischen den detektierten Vitalsignalen und den Stress- und Burnout-Vitalmustern vorliegt.

Weiterhin wird gleichzeitig durch das Analyse- und Diagnosegerät 1 mittels des Hautimpendanzsensors 4 die Leitfähigkeit der Haut des Probanden gemessen, welche immer dann ansteigt, wenn der Proband in eine Stresssituation gerät. Besonders häufig auftretende Stresssituationen, und damit besonders häufige auftretende hohe Leitfähigkeit der Haut, lassen bereits auf das Vorliegen von Stress und/oder des Burnout-Syndroms schließen. Auch hier wird ein zeitaufgelöster Verlauf der mit dem Hautimpendanzsensor 4 detektierten Vitalsignale in der Auswerteeinheit festgehalten, welcher mit zeitaufgelösten Stress- und Burnout-Vitalmustern betreffend die Hautimpendanz verglichen wird. Sind auch hier Übereinstimmungen zwischen den detektierten Vitalsignalen und den Stress- und Burnout-Vitalmustern aufzufinden, so erkennt die Auswerteeinheit, dass Stress und/oder ein Burnout-Syndrom vorliegt.

Es ist also mit dem Gerät ein Bündel an Vitalsignalen auswertbar, die Stress und/oder das Burnout-Syndrom sehr eindeutig identifizieren. Die Anzeigevorrichtung 7 gibt dem Probanden am Ende der Messung das Diagnoseergebnis der Auswerteeinheit aus. Im vorliegenden Ausführungsbeispiel bekommt der Proband wie bei einer Ampel den Hinweis, ob Stress und/oder ein Burnout-Syndrom vorliegt (rotes Licht), ob der Proband gefährdet ist, am Burnout-Syndrom zu erkranken (gelbes Licht), oder ob eine Gefährdung gänzlich auszuschließen ist (grünes Licht).

### Bezugszeichenliste

- 1: Analyse- und Diagnosegerät
- 2: Sensor
- 3: Bioakustischer Sensor
- 4: Hautimpendanzsensor
- 5: Sensoranordnung
- 6: Bedienelement
- 7: Anzeigevorrichtung
- 8: Schnittstelle
- 9: Gurtsystem

## Patentansprüche

1. Analyse- und Diagnosegerät (1) zur Detektion von Stress bei einem Probanden mit mindestens einem Sensor (2), welcher ausgebildet ist Vitalsignale des Probanden zu detektiere und diese einer Speichereinheit zuzuführen einer Spannungsversorgung, sowie mit einer mit der Speichereinheit verbundenen Auswerteeinheit, wobei mittels der Auswerteeinheit ein zeitaufgelöster Verlauf der durch den mindestens einen Sensor (2) detektierten Vitalsignale auswertbar ist, und wobei in der Auswerteeinheit ein Komparator vorgesehen ist, welcher ausgebildet ist mittels eines Abgleichs des zeitaufgelösten Verlaufs der detektierten Vitalsignale mit in einer Datenbank gespeicherten zeitaufgelösten Stress-Vitalmustern Stress zu diagnostiziere, **dadurch gekennzeichnet, dass** der Sensor (2) als ein bioakustischer Sensor (3) zur Detektion intrakorporal erzeugten Schalls ausgebildet ist, wobei weiterhin eine Alarmeinheit vorgesehen ist zur Alarmierung des Probanden oder Patienten im Falle einer Stresssituation.

2. Analyse- und Diagnosegerät (1) nach Anspruch 1, **dadurch gekennzeichnet, dass** die die Stress- Vitalmuster enthaltende Datenbank in der Speichereinheit vorgesehen ist.

3. Analyse- und Diagnosegerät (1) nach Anspruch 1, **dadurch gekennzeichnet, dass** die die Stress- Vitalmuster enthaltende Datenbank in einem externen, über ein Netzwerk zugänglichen Rechen- und Speicherwerk vorgesehen ist.

4. Analyse- und Diagnosegerät (1) nach einem der Ansprüche 1 bis 3, **dadurch gekennzeichnet, dass** eine Sensoranordung (5) vorgesehen ist, die den bioakustischen Sensor (3), sowie mindestens einen weiteren Sensor (2) aufweist, der aus der Gruppe bestehend aus einem Gasflusssensor zur Messung des Gasflusses der Atmung, einem bioakustischen Sensor (3) zur Messung der Probandengeräusche, einem Hautimpendanzsensor (4), einem EKG-Sensor, einem Pulsoximetriesensor, einem EOG-Sensor und einer Einheit zur Messung des Blutdrucks ausgewählt ist.

5. Analyse- und Diagnosegerät (1) nach einem der Ansprüche 1 bis 4, **dadurch gekennzeichnet, dass** mindestens ein Bedienelement (6) vorgesehen ist, welches vorzugsweise als ein Einknopfbedienelement gebildet ist.

6. Analyse- und Diagnosegerät (1) nach einem der Ansprüche 1 bis 5, **dadurch gekennzeichnet, dass** eine Anzeigevorrichtung (7) vorgesehen ist, und dass die Anzeigevorrichtung (7) geeignet ist, das Diagnoseergebnis der Auswerteeinheit darzustellen.

7. Analyse- und Diagnosegerät (1) nach Anspruch 6, **dadurch gekennzeichnet, dass** die Anzeigevorrichtung (7) gleichwohl als ein Eingabeterminal gebildet ist, das vorzugsweise als Tastschirm gebildet ist.

8. Analyse- und Diagnosegerät (1) nach einem der Ansprüche 1 bis 7, **dadurch gekennzeichnet, dass** mindestens eine Schnittstelle (8) zur Dateneingabe und/oder zur Datenausgabe vorgesehen ist.

9. Analyse- und Diagnosegerät (1) nach einem der Ansprüche 1 bis 8, **dadurch gekennzeichnet, dass** die Auswerteeinheit als ein Bauteil gebildet ist, welches aus der Gruppe bestehend aus einem Mikrocontroller, einem ASIC, einem FPAA, und einem FPGA ausgewählt ist.

10. Analyse- und Diagnosegerät (1) nach Anspruch 9, dadurch gekennzeicht, dass dem Mikrokontroller mindestens ein Frequenzfiltersystem zugeordnet ist.

11. Analyse- und Diagnosegerät (1) nach einem der Ansprüche 1 bis 10, **dadurch gekennzeichnet, dass** ein mit der Auswerteeinheit verbundener interner oder externer Analog-Digital-Wandler vorgesehen ist.

12. Analyse- und Diagnosegerät (1) nach einem der Ansprüche 1 bis 11, **dadurch gekennzeichnet, dass** ein Gurtsystem (9) zur Lagefixierung des mindestens einen Sensors (3) vorgesehen ist.

13. Analyse- und Diagnosegerät (1) nach Anspruch 12, **dadurch gekennzeichnet, dass** der mindestens eine Sensor (3) in das Gurtsystem (9) integriert ist.

14. Analyse- und Diagnosegerät (1) nach Anspruch 12 oder 13, **dadurch gekennzeichnet, dass** das Gurtsystem (9) aus textilem Material, vorzugsweise elastisch gebildet ist.

## Claims

1. An analysis and diagnostic device (1) for detecting stress in an experimentee comprising at least one sensor (2) adapted to detect vital signals of the experimentee and to feed same to a memory unit, a voltage supply, and an evaluation unit connected to the memory unit, wherein a time-resolved configuration of the vital signals detected by the at least one sensor (2) can be evaluated by means of the evaluation unit and wherein provided in the evaluation unit is a comparator adapted to diagnose stress by means of a comparison of the time-resolved configuration of the detected vital signals with time-resolved stress vital patterns stored in a data base, **characterised in that** the sensor (2) is in the form of a bioacoustic sensor (3) for the detection of intracoporeally produced sound, wherein there is further provided an alarm unit for alarming the experimentee or patient in the case of a stress situation.

2. An analysis and diagnostic device (1) according to claim 1 **characterised in that** the database containing the stress vital patterns is provided in the memory unit.

3. An analysis and diagnostic device (1) according to claim 1 **characterised in that** the database containing the stress vital patterns is provided in an external computing and memory system accessible by way of a network.

4. An analysis and diagnostic device (1) according to one of claims 1 to 3 **characterised in that** there is provided a sensor arrangement (5) having the bioacoustic sensor (3) and at least one further sensor (2) selected from the group comprising a gas flow sensor for measuring the gas flow of respiration, a bioacoustic sensor (3) for measuring the experimentee noises, a skin impedance sensor (4), an ECG sensor, a pulse oximeter sensor, an EOG sensor and a unit for measuring blood pressure.

5. An analysis and diagnostic device (1) according to one of claims 1 to 4 **characterised in that** there is provided at least one operating element (6) which is preferably in the form of a single-knob operating element.

6. An analysis and diagnostic device (1) according to one of claims 1 to 5 **characterised in that** there is provided a display device (7) and the display device (7) is suitable for representing the diagnosis result of the evaluation unit.

7. An analysis and diagnostic device (1) according to claim 6 **characterised in that** the display device (7) is nonetheless in the form of an input terminal which is preferably in the form of a touch screen.

8. An analysis and diagnostic device (1) according to one of claims 1 to 7 **characterised in that** there is provided at least one interface (8) for data input and/or for data output.

9. An analysis and diagnostic device (1) according to one of claims 1 to 8 **characterised in that** the evaluation unit is in the form of a component selected from the group comprising a microcontroller, an ASIC, an FPAA and an FPGA.

10. An analysis and diagnostic device (1) according to claim 9 **characterised in that** at least one frequency filter system is associated with the microcontroller.

11. An analysis and diagnostic device (1) according to one of claims 1 to 10 **characterised in that** there is provided an internal or external analog-digital converter connected to the evaluation unit.

12. An analysis and diagnostic device (1) according to one of claims 1 to 11 **characterised in that** there is provided a belt system (9) for fixing the at least one sensor (3) in position.

13. An analysis and diagnostic device (1) according to claim 12 **characterised in that** the at least one sensor (3) is integrated into the belt system (9).

14. An analysis and diagnostic device (1) according to claim 12 or claim 13 **characterised in that** the belt system (9) is formed from textile material, preferably elastic.

## Revendications

1. Appareil d'analyse et de diagnostic (1) destiné à détecter le stress chez une personne participant à une étude, comprenant au moins un capteur (2) qui est conçu pour détecter des signaux vitaux de la personne participant à l'étude et pour acheminer ceux-ci à une unité de mémoire, comprenant une alimentation en tension ainsi qu'une unité d'évaluation reliée à l'unité de mémoire, l'unité d'évaluation permettant d'exploiter une révolution solution temporelle des signaux vitaux détectés par le capteur (2), au nombre d'au moins un, et l'unité d'évaluation comportant un comparateur qui est conçu pour diagnostiquer le stress à l'aide d'un rapprochement de l'évolution à résolution temporelle des signaux vitaux détectés avec des modèles vitaux de stress à résolution temporelle stockés dans une base de données, **caractérisé en ce que** le capteur (2) est réalisé sous la forme d'un capteur bioacoustique (3) en vue de la détection de sons produits de façon intracorporelle, sachant qu'en outre il est prévu une unité d'alarme destinée à alerter la personne participant à l'étude ou le patient, en cas de situation de stress.

2. Appareil d'analyse et de diagnostic (1) selon la revendication 1, **caractérisé en ce que** la base de données contenant les modèles vitaux de stress est prévue dans l'unité de mémoire.

3. Appareil d'analyse et de diagnostic (1) selon la revendication 1, **caractérisé en ce que** la base de données contentant les modèles vitaux de stress est prévue dans une unité de calcul et de mémoire externe, accessible par l'intermédiaire d'un réseau.

4. Appareil d'analyse et de diagnostic (1) selon l'une des revendications 1 à 3, **caractérisé en ce qu'**il est prévu un ensemble de capteurs (5) qui comprend le capteur biacoustique (3) ainsi qu'au moins un autre capteur (2) qui est sélectionné dans le groupe comprenant un capteur de flux gazeux, destiné à mesurer le flux gazeux de la respiration, un capteur bioacoustique (3), destiné à mesurer les sons émis par la personne participant à l'étude, un capteur d'impédance de la peau (4), un capteur d'électrocardiogramme, un capteur oxymétrique du pouls, un capteur EOG et une unité destinée à mesurer la tension artérielle.

5. Appareil d'analyse et de diagnostic (1) selon l'une des revendications 1 à 4, **caractérisé en ce qu'**il est prévu au moins un élément de commande (6) qui est de préférence réalisé sous la forme d'un élément de commande à i7auton unique.

6. Appareil d'analyse et de diagnostic (1) selon l'une des revendications 1 à 5, **caractérisé en ce qu'**il est prévu un dispositif d'affichage (7), et **en ce que** le dispositif d'affichage (7) est adapté pour représenter le résultat de diagnostic de l'unité d'évaluation.

7. Appareil d'analyse et de diagnostic (1) selon la revendication 6, **caractérisé en ce que** le dispositif d'affichage (7) est en même temps réalisé comme terminal d'entrée qui est réalisé de préférence sous forme d'écran tactile.

8. Appareil d'analyse et de diagnostic (1) selon l'une des revendications 1 à 7, **caractérisé en ce qu'**il est prévu au moins une interface (8) destinée à l'entrée de données et/ou à la sortie de données.

9. Appareil d'analyse et de diagnostic (1) selon l'une des revendications 1 à 8, **caractérisé en ce que** l'unité d'évaluation est réalisée sous la forme d'un composant qui est sélectionné dans le groupe comprenant un microcontrôleur, un ASIC, un FPAA et un FPGA.

10. Appareil d'analyse et de diagnostic (1) selon la revendication 9, **caractérisé en ce qu'**au moins un système de filtrage en fréquence est associé au microcontrôleur.

11. Appareil d'analyse et de diagnostic (1) selon l'une des revendications 1 à 10, **caractérisé en ce qu'**il est prévu un convertisseur analogique-numérique interne ou externe qui est relié à l'unité d'évaluation.

12. Appareil d'analyse et de diagnostic (1) selon l'une des revendications 1 à 11, **caractérisé en ce qu'**il est prévu un système de sangle (9) destiné à maintenir la position du capteur (3), au nombre d'au moins un.

13. Appareil d'analyse et de diagnostic (1) selon la revendication 12, **caractérisé en ce que** le capteur (3), au nombre d'au moins un, est intégré dans le système de sangle (9).

14. Appareil d'analyse et de diagnostic (1) selon la revendication 12 ou 13, **caractérisé en ce que** le système de sangle (9) est réalisé dans une matière textile, de préférence de façon élastique.
